# EUROPEAN PATENT APPLICATION

(11) **EP 1 997 503 A2**
(43) Date of publication of application: **03.12.2008**
(21) Application number: 07019462.6
(22) Date of filing: 04.10.2007
(51) Int. Cl.: A61K 38/00, A61P 35/00, A61P 35/04

(54) **Treatment of melanoma with alpha thymosin peptides**

(30) Priority: 01.06.2007 US 941467 P; 03.07.2007 US 947802 P; 20.09.2007 US 858640
(71) Applicant: SciClone Pharmaceuticals, Inc., San Mateo CA 94404 (US)
(72) Inventor: Tuthill, Cynthia W., Menlo Park CA 94025 (US); Rios, Israel, Menlo Park CA 94025 (US)
(74) Representative: Höhfeld, Jochen

(57) **Abstract**

A method of treating melanoma or a metastasis thereof in a human patient by administering a melanoma-treating effective amount of an alpha thymosin peptide to a human melanoma patient, wherein the human melanoma patient does not have a substantially elevated LDH blood level.

## Description

### CROSS-REFERENCE TO RELATED APPLICATION

This application claims the benefit of U.S. Provisional Application Serial No. 60/941,467, filed June 1, 2007, and U.S. Provisional Application Serial No. 60/947,802, filed July 3, 2007, which are both incorporated herein.

### FIELD OF THE INVENTION

The present invention relates to the field of melanoma treatment.

### BACKGROUND OF THE INVENTION

Skin cancer is the most common form of cancer in the United States. In 2007, The American Cancer Society estimates that approximately 8,110 deaths will occur from melanoma and another 59,940 cases of melanoma are expected to be diagnosed in this country.

Melanoma is a malignant tumor of melanocytes which are found predominantly in skin but also in bowel and the eye (uveal melanoma). It is one of the rarer types of skin cancer but causes the majority of skin cancer related deaths.

The treatment includes surgical removal of the tumor; adjuvant treatment; chemo- and immunotherapy, or radiation therapy. Of particular danger are metastases of the primary melanoma tumor.

Melanoma is classified as stage IV, the most advanced form, once the cancer has spread beyond the skin to a distant site. DTIC and interleukin-2 (IL-2) are the only FDA-approved therapies for the treatment of malignant melanoma. However, other therapeutic agents including alpha interferon, used alone or in combination, are ineffective at extending overall patient survival, which at this stage is typically only about six to nine months. Response to treatment largely depends upon the stage of melanoma, disease site and the extent to which the cancer has spread.

There remains a need in the art for improved treatments of melanoma.

### SUMMARY OF THE INVENTION

In accordance with the present invention, a method of treating melanoma or a metastasis thereof in a human patient comprises administering a melanoma-treating effective amount of an alpha thymosin peptide to a human melanoma patient, during a treatment regimen wherein the human melanoma patient does not have a substantially elevated LDH blood level.

### BRIEF DESCRIPTION OF THE DRAWINGS

Figs. 1A and 1B graphically show overall survival in a patient population with various melanoma treatments.

Figs. 2A and 2B graphically show overall survival in normal LDH patients with various melanoma treatments.

Figs. 3A and 3B graphically show progression-free survival in a patient population with various melanoma treatments.

Figs. 4A and 4B graphically show progression-free survival in normal LDH patients with various melanoma treatments.

### DESCRIPTION OF THE PREFERRED EMBODIMENTS

The present invention is directed to a method of treating melanoma or metastases thereof in human patients. The method involves administering melanoma-treating effective amounts of an alpha thymosin peptide to human melanoma patients which do not have substantially elevated lactate dehydrogenase (LDH) blood levels.

It surprisingly has been found that human metastatic melanoma patients without substantially elevated LDH blood levels, when treated according to the present invention, have an overall duration of survival that is dose-responsive to alpha thymosin peptide even when tumor response is not dose-responsive.

In one embodiment, the method comprises first measuring LDH blood level in the patient, then determining if said LDH blood level is not elevated, and if said LDH blood level is not elevated, then administering said alpha thymosin peptide to said patient in the treatment regimen

Alpha thymosin peptides comprise thymosin alpha 1 (TA1) peptides including naturally occurring TA1 as well as synthetic TA1 and recombinant TA1 having the amino acid sequence of naturally occurring TA1, amino acid sequences substantially similar thereto, or an abbreviated sequence form thereof, and their biologically active analogs having substituted, deleted, elongated, replaced, or otherwise modified sequences which possess bioactivity substantially similar to that of TA1, e.g., a TA1 derived peptide having sufficient amino acid homology with TA1 such that it functions in substantially the same way with substantially the same activity as TA1. Suitable dosages of the alpha thymosin peptide can be within the range of about 0.001-10mg/kg/day.

The terms "thymosin alpha 1" and "TA1" refer to peptides having the amino acid sequence disclosed in U.S. patent number 4,079,137, the disclosure of which is incorporated herein by reference.

Thymosin alpha 1 (TA1), initially isolated from Thymosin Fraction 5 (TF5), has been sequenced and chemically synthesized. TA1 is a 28 amino acid peptide with a molecular weight of 3108.

Effective amounts of an alpha thymosin peptide are amounts which may be dosage units within a range corresponding to about 0.1-20 mg of TA1, preferably 1-10 mg of TA1, more preferably about 2-10 mg of TA1, still more preferably 2-7 mg of TA1, and most preferably, the dosage unit is within a range of 3-6.5 mg, and comprises about 1.6, 3.2 or 6.4 mg of TA1, most preferably about 3.2 or 6.4 mg of TA1. A dosage unit may be administered once per day, or a plurality of times per day.

Melanoma has various stages, which may include Stage 0, I, II, III and IV, as well as their respective subdivisions. In certain embodiments, the melanoma being treated is malignant metastatic melanoma. In certain embodiments, the melanoma being treated is stage I, stage II, stage III or stage IV. In other embodiments, the melanoma being treated is stage M1a, M1b or M1c melanoma.

In preferred embodiments, the alpha thymosin peptide is administered in a treatment regimen which substantially excludes administration to the patient of an immune-stimulating cytokine, such as immune-stimulating interferons including interferon alpha (IFN alpha) and interferon beta (IFN beta), and/or immune-stimulating interleukins such as Interleukin 2 (IL-2). In some embodiments, the treatment regimen substantially excludes administration to the patient of any immune-stimulating cytokine. In certain embodiments, the treatment regimen substantially excludes administration to the patient of any immune-stimulating interferon. In some embodiments, the treatment regimen substantially excludes administration to the patient of any immune-stimulating interleukin. In further embodiments, the method excludes administration to the patient of IFN alpha, IFN beta, IL-2 or combinations thereof. In still further embodiments, the treatment regimen substantially excludes administration of a cytokine as set forth herein to a patient during the treatment in an amount significant to treatment of melanoma.

It surprisingly has been found that patients treated in a treatment regimen with TA1 but without IFN alpha, showed a higher overall tumor response, a higher median survival rate, and a higher progression-free survival, than patients treated with both IFN alpha and TA1.

In preferred embodiments, the melanoma patient being treated has an LDH blood level below about 475 IU/L, below about 450 IU/L, below about 435 IU/L, below about 400 IU/L, or below about 365 IU/L. In some embodiments, the melanoma patient has an LDH blood level of between about 100-335 IU/L. In further embodiments, the melanoma patient being treated has an LDH blood level which is substantially within a normal human range, e.g., between about 150-335 IU/L.

In some embodiments, a patient's LDH blood level is below 1.3 times the upper limit of the normal range, below 1.2 times the upper limit of the normal range or below 1.1 times the upper limit of the normal range.

In certain embodiments, the method of the present invention comprises administering the alpha thymosin peptide along with administering another anti-neoplastic agent other than the alpha thymosin peptide, during a course of the treatment regimen. The other neoplastic agent may be administered concurrently with the alpha thymosin peptide or separately therefrom during the treatment regimen, e.g., on the same day(s) as the alpha thymosin peptide or on different days during the course of the treatment regimen. In preferred embodiments, the other anti-neoplastic agent is an alkylating anti-neoplastic agent, such as dacarbazine (DTIC) which may be administered in a dosage range of, e.g., 700-1300 mg/m² patient body surface area per day, more preferably 800-1200 mg/m², most preferably 1000 mg/m².

In preferred embodiments, the treatment regimen comprises a plurality of days, with the alpha thymosin peptide comprising thymosin alpha 1 (TA1), and the TA1 being administered to the patient during at least a portion of the treatment regimen at a dosage within a range of about 0.5-10 mg/day. In certain embodiments, the dosage is within a range of about 1.5-7 mg/day, or within a range of about 1.6-6.4 mg/day. In certain preferred embodiments, the dosage is within a range of 1.7-10 mg/day, more preferably 1.7-7 mg/day, still more preferably 3-7 mg/day. Exemplary dosages include 1.6, 3.2 and 6.4 mg/day.

In preferred embodiments, the treatment regimen comprises administering the alpha thymosin peptide for a period of about 1-10 days, followed by about 1-5 days of non-administration of the alpha thymosin peptide. More preferably, the alpha thymosin peptide is administered daily for about 3-5 days, followed by about 2-4 days of non-administration of the alpha thymosin peptide. Still more preferably, the alpha thymosin peptide is administered daily for about 4 days, followed by about 3 days of non-administration of the alpha thymosin peptide.

In a particularly preferred embodiment of the present invention, an alkylating anti-neoplastic agent such as DTIC is administered to the patient at the beginning of a treatment regimen about 7 days prior to administration of the alpha thymosin peptide to the patient.

In certain embodiments, the DTIC is administered to the patient intravenously at a dosage of about 1000 mg/m² patient surface area.

In one preferred embodiment, a treatment regimen comprises administration of an alkylating anti-neoplastic agent, such as DTIC, on a first day of treatment, followed by about 6 consecutive days of non-administration of the anti-neoplastic agent to the patient, followed by about 4 consecutive days of administration of the alpha thymosin peptide to the patient, followed by about 3 consecutive days of non-administration of the anti-neoplastic agent and the alpha thymosin peptide to the patient, followed by about 4 consecutive days of further administration to the patient of the alpha thymosin peptide. In particularly preferred embodiments, this treatment regimen is repeated a plurality of times with the patient, with each subsequent treatment regimen being commenced within about 7-35 days from the end of a prior treatment regimen, more preferably within about 21-28 days of the end of a prior treatment regimen.

It surprisingly has been discovered that an alpha thymosin peptide is able to up-regulate (enhance expression of) melanoma-specific antigens, including melan-A and MART-1 on cell surfaces of melanoma cells, including mouse B-16 melanoma cells. Thus, the invention also is applicable to up-regulation (enhancing expression of) melanoma-specific antigens on cell surfaces of melanoma cells.

According to one embodiment, the invention comprises use of an alpha thymosin peptide in a melanoma-treating effective amount in manufacture of a medicament for use in a treatment regimen for treating melanoma or a metastasis thereof in a human melanoma patient who does not have a substantially elevated LDH blood level.

According to one embodiment, the medicament is for use in a treatment regimen which substantially excludes administration to the patient of interferon α (IFN alpha), interferon β (IFN beta), Interleukin 2 (IL-2), or a combination thereof.

According to one embodiment, said medicament is for use in a treatment regimen which substantially excludes any immune-stimulating cytokine to said patient during said treatment regimen in an amount significant for treatment of melanoma or a metastasis thereof.

According to one embodiment, said LDH blood level is below 475 IU/L.

According to one embodiment, said LDH blood level is between 100 - 335 IU/L.

One embodiment is the manufacture of a pharmaceutical combination including said alpha thymosin peptide, said combination further comprising an anti-neoplastic agent other than said alpha thymosin peptide for use during a course of the treatment regimen, which alpha thymosin peptide and other anti-neoplastic agent may be administered separately or together.

According to one embodiment, said anti-neoplastic agent is an alkylating anti-neoplastic agent.

According to one embodiment, said anti-neoplastic agent is dacarbazine (DTIC).

According to one embodiment, said medicament is for use in a treatment regimen which comprises a plurality of days, said alpha thymosin peptide comprises thymosin alpha 1 (TA1), and said TA1 is for use in administration to said patient during at least a portion of said treatment regimen at a dosage within a range of 0.5-10 mg/day.

According to one embodiment, said dosage is within a range of 1.5-7 mg/day.

According to one embodiment, said dosage is 3.2 mg/day.

According to one embodiment, said dosage is 6.4 mg/day.

According to one embodiment, said alpha thymosin peptide is TA1 and said medicament is for use in a treatment regimen which comprises administration of TA1 daily for a period of about 1-10 days, followed by about 1-5 days of non-administration of said TA1.

According to one embodiment, said TA1 is for use in administration daily for about 3-5 days, followed by about 2-4 days of non-administration of said TA1.

According to one embodiment, said TA1 is for use in administration daily for about 4 days, followed by about 3 days non-administration of said TA1.

According to one embodiment, said dacarbazine is for administration to said patient about 7 days prior to administration of said TA1 to said patient.

According to one embodiment, said dacarbazine is at a dosage of 700-1300 mg/m².

According to one embodiment, said dacarbazine is at a dosage of 1000 mg/m².

One embodiment is for use in a treatment regimen which comprises administration of dacarbazine (DTIC) on a first day of treatment, followed by 6 consecutive days of non-administration of any anti-neoplastic agent to said patient, followed by 4 consecutive days of administration of TA1 to said patient, followed by 3 consecutive days of non-administration of the DTIC and TA1 to said patient, followed by 4 consecutive days of administration to said patient of TA1, and which treatment regimen further comprises repeating said treatment regimen a plurality of times with said patient, with each subsequent treatment regimen being commenced within 7-35 days from an end of a prior treatment regimen.

According to one embodiment, each said subsequent treatment regimen is commenced within 21-28 days of the end of a prior treatment regimen.

According to one embodiment, the alpha thymosin peptide enhances melanoma-specific surface antigens on melanoma cells.

According to one embodiment, said antigens are melan-A, MART-1 or a combination thereof.

According to one embodiment, the dosage range of said TA1 is within a range of 3-7 mg/day.

The invention is illustrated by the followed example, which is not intended to be limiting.

Example 1

This phase 2 multi-center, open-label study enrolled 488 patients with stage IV metastatic melanoma at 64 European clinical sites. The trial was designed to evaluate different dose levels of Thymosin alpha 1 (TA1). This study was in combination with DTIC chemotherapy, with and without low-dose interferon alpha, as a first-line treatment for malignant melanoma. Most patients enrolled in the trial had liver and other metastases and the remaining patients had lung metastases and skin or lymph node metastases. Thymosin alpha 1 at all dose levels was well-tolerated in all treated patients, with no serious adverse events attributed to the drug.

A total of 488 patients (pts) (63% M1c; 24% M1 b; 13% M1a) were randomised into the study from 64 European sites. Demographic and baseline characteristics are shown in table 1 wherein DIT=DTIC/IFN alpha/TA1, DT=DTIC/TA1, T=TA1, DI=DTIC/IFN alpha and ECOG PS is a patient's health performance status (PS) according to the Eastern Cooperative Oncology Group (ECOG).

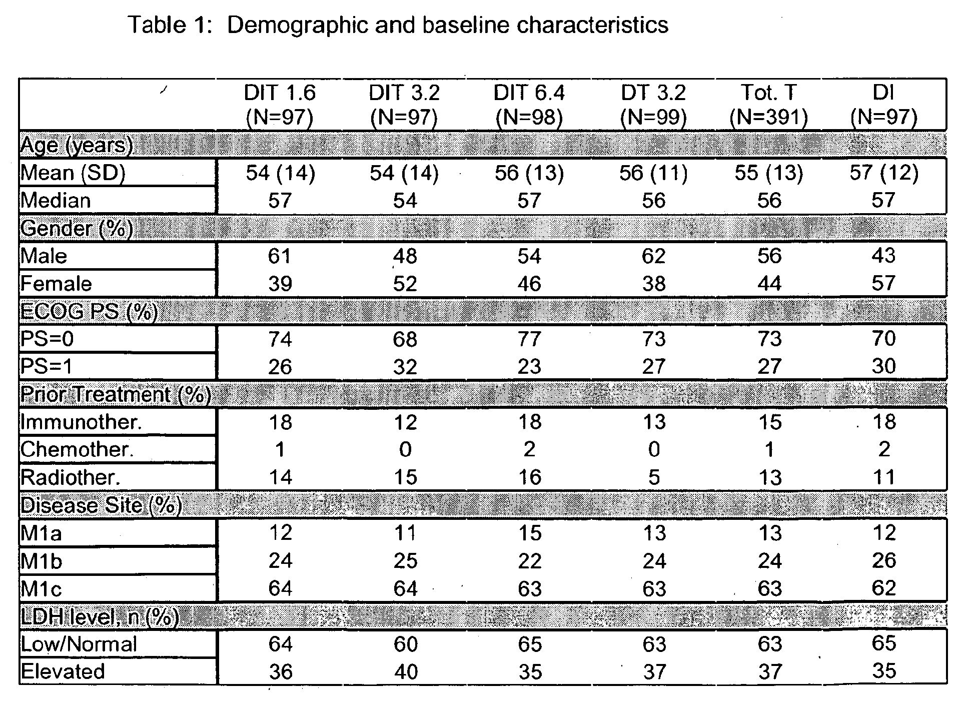

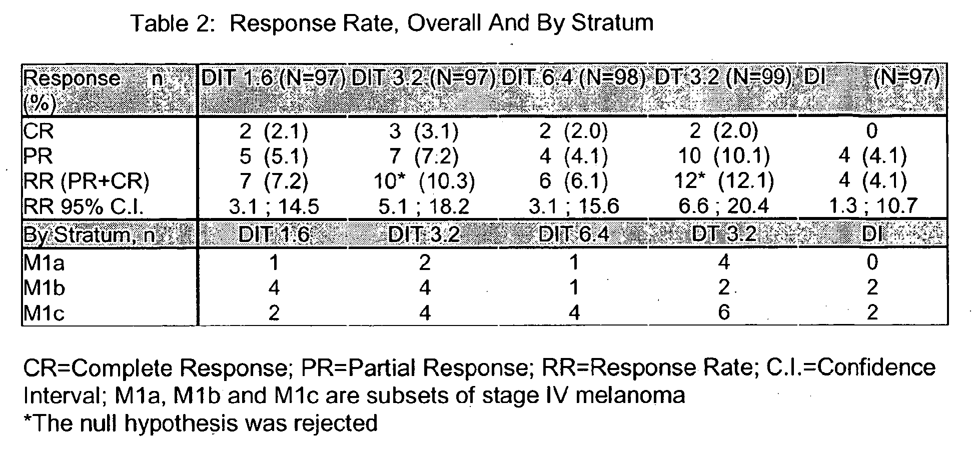

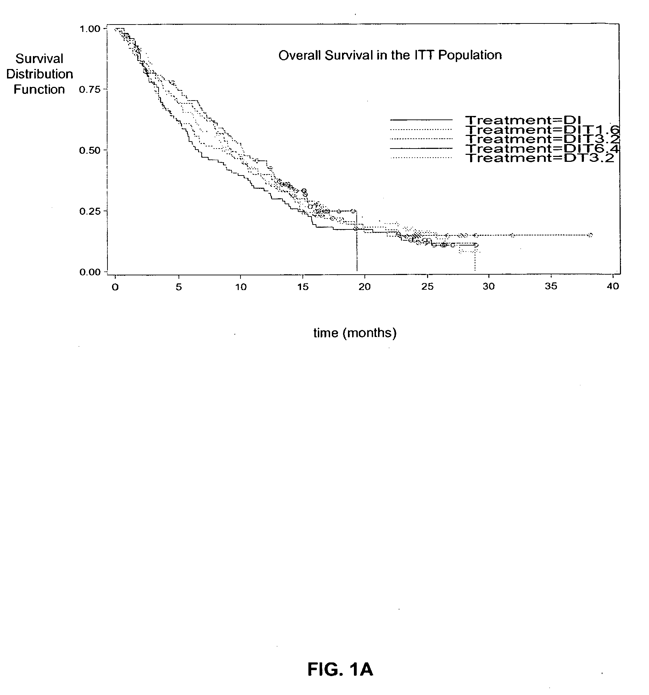

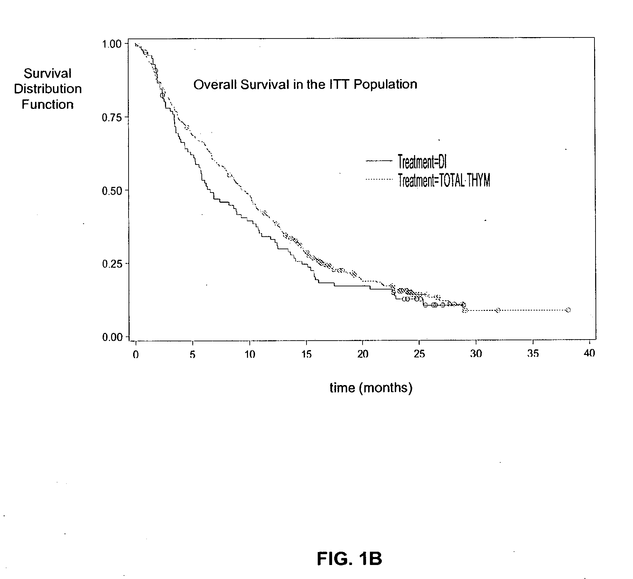

**Table 3: Shows data on overall survival (OS) of the intent to treat (ITT) population graphically depicted in Figs. 1A and 1B**

| OS (months) | DIT 1.6 | DIT 3.2 | DIT 6.4 | DT 3.2 | Tot T | DI |
|---|---|---|---|---|---|---|
| Median | 9.3 | 8.5 | 10.2 | 9.3 | 9.4 | 6.6 |
| 95% Cl | 7.9 ; 11.0 | 6.1; 11.4 | 8.2 ; 12.6 | 6.7; 11.5 | 8.3 ; 10.5 | 5.2 ; 9.8 |

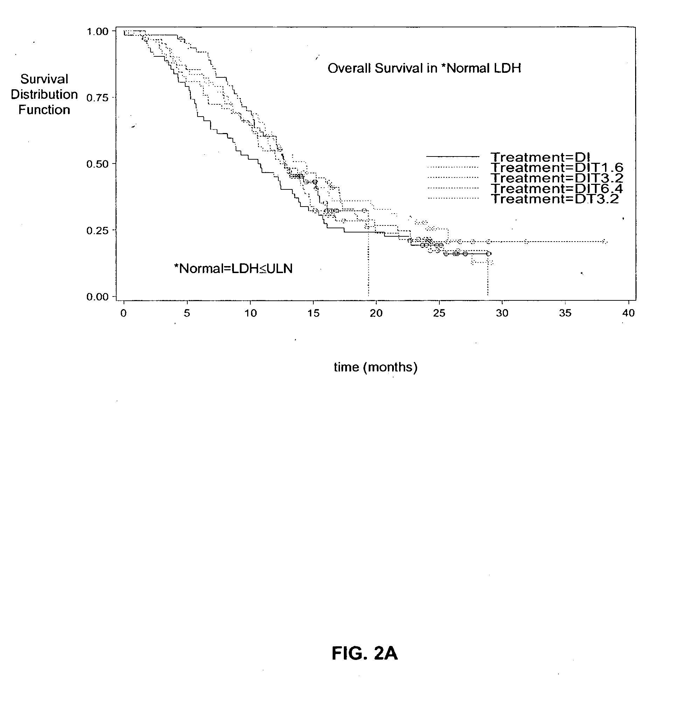

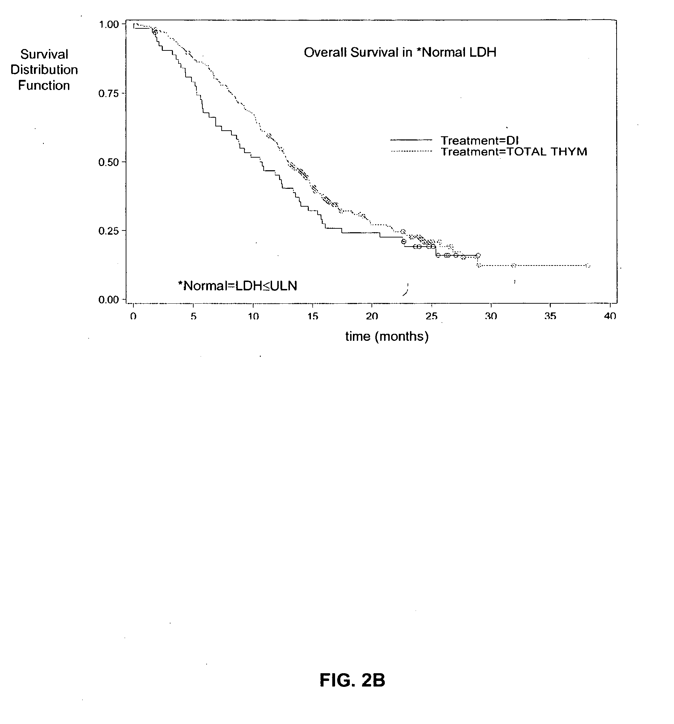

**Table 4: Shows data on OS in normal LDH patients as graphically depicted in Figs. 2A and 2B**

| OS (months) | DIT 1.6 | DIT 3.2 | DIT 6.4 | DT 3.2 | Tot T | DI |
|---|---|---|---|---|---|---|
| Median | 12.9 | 12.6 | 12.8 | 14.4 | 12.9 | 10.8 |
| 95% Cl | 10.2 ; 11.0 | 10.1; 17.1 | 10.8 ; 15.5 | 11.7 ; 16.3 | 12.1 ; 14.5 | 7.4 ; 13.6 |

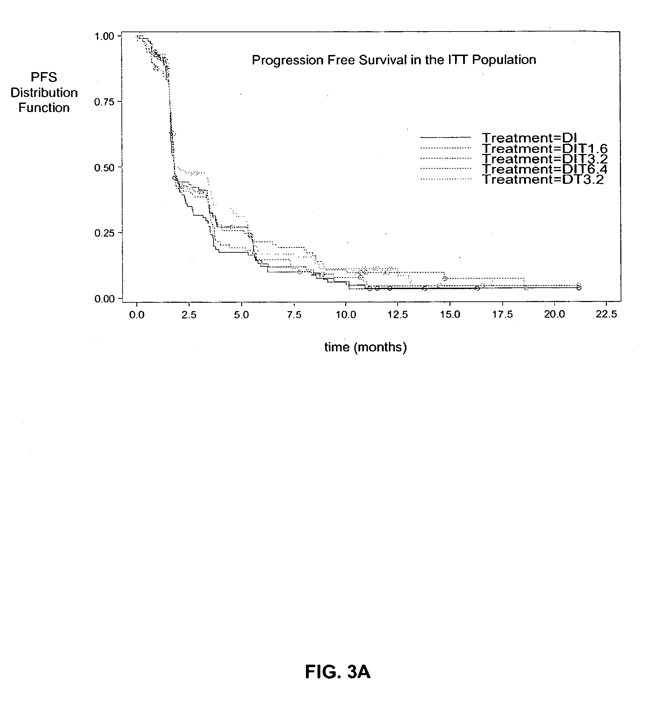

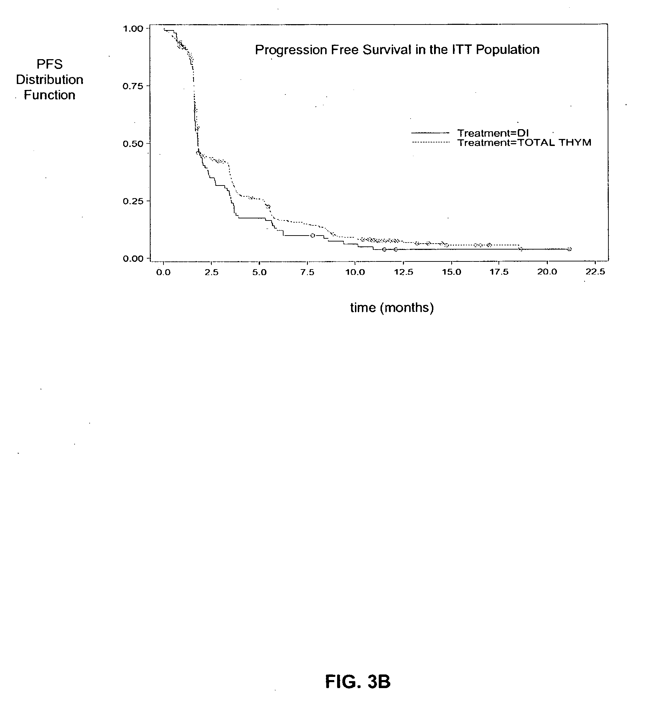

**Table 5: Shows data on progression free survival (PFS) in the ITT population shown in Figs. 3A and 3B.**

| PFS (months) | DIT 1.6 | DIT 3.2 | DIT 6.4 | DT 3.2 | Tot T | DI |
|---|---|---|---|---|---|---|
| Median | 1.84 | 1.84 | 1.84 | 1.87 | 1.84 | 1.81 |
| 95% Cl | 1.74 ; 3.22 | 1.74; 2.60 | 1.74 ; 3.38 | 1.81 ; 3.58 | 1.81 ; 2.07 | 1.64 ; 2.17 |

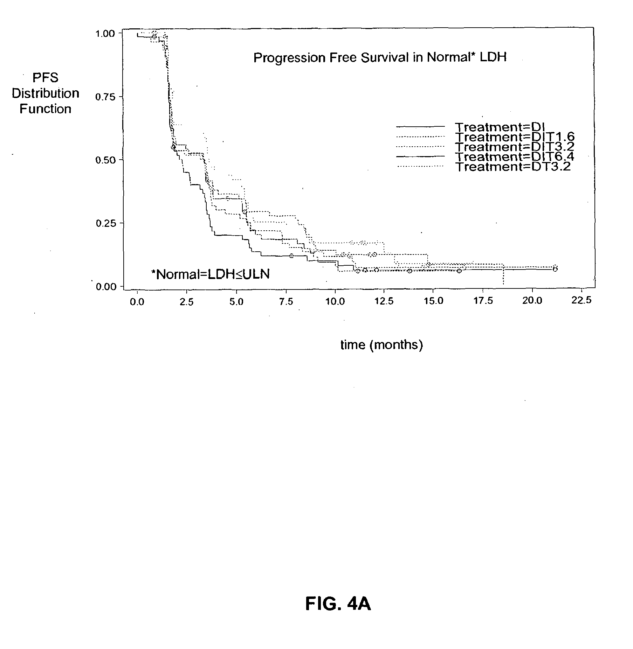

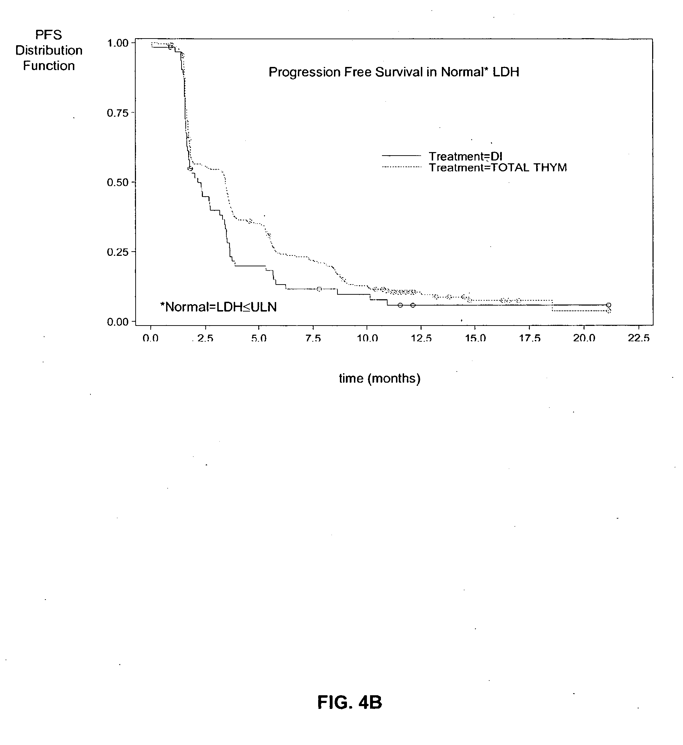

**Table 6: Shows PFS in normal LDH patients shown in Figs. 4A and 4B**

| PFS (months) | DIT 1.6 | DIT 3.2 | DIT 6.4 | DT 3.2 | Tot T | DI |
|---|---|---|---|---|---|---|
| Median | 3.33 | 3.38 | 3.38 | 3.65 | 3.45 | 2.17 |
| 95% Cl | 1.77; 3.71 | 1.77; 4.07 | 1.84 ; 3.78 | 2.33 ; 5.42 | 2.33 ; 3.61 | 1.71 ; 3.31 |

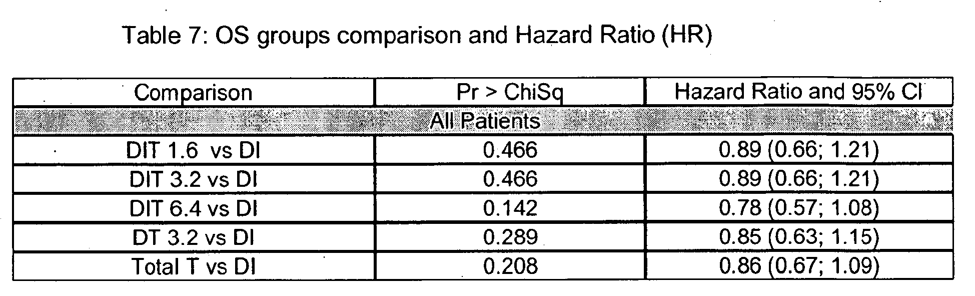

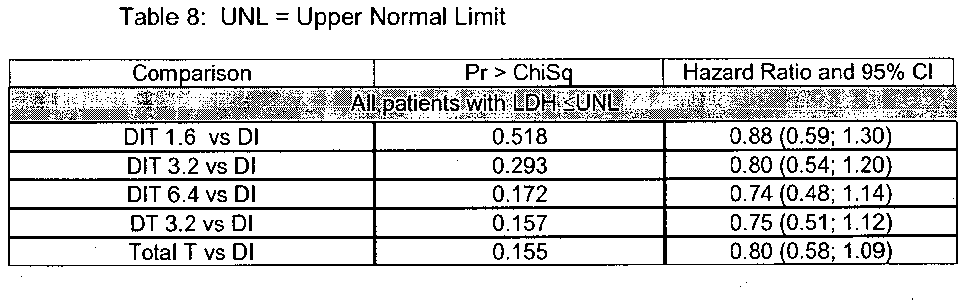

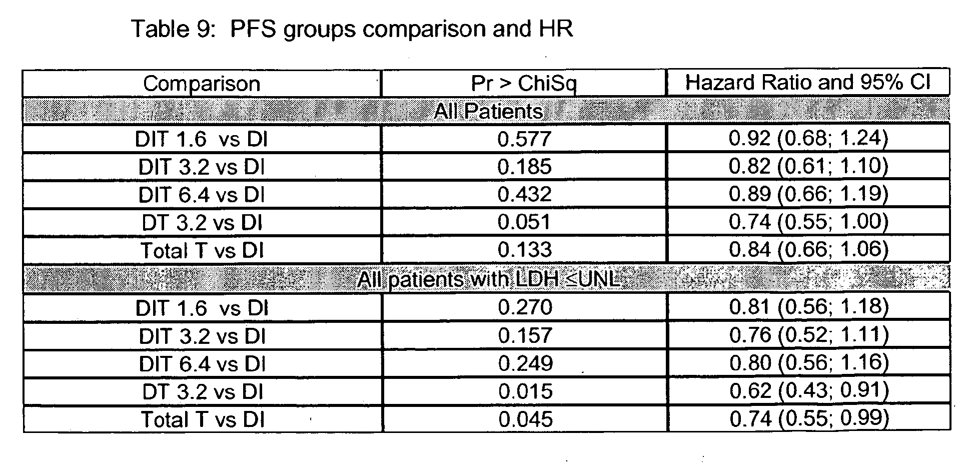

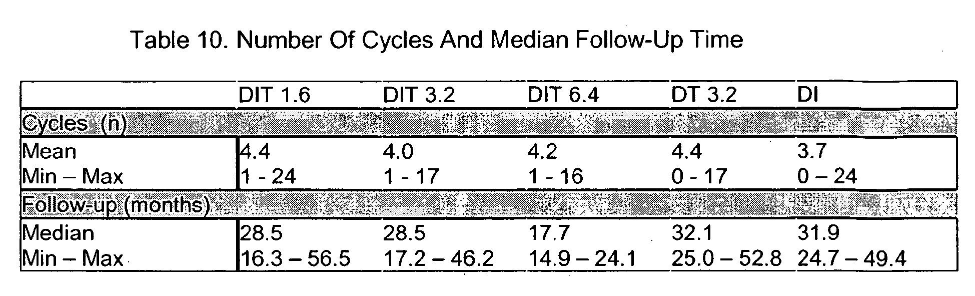

**Table 11. Number of Patients With Serious And Not Serious Adverse Events (AEs)**

| | DIT 1.6 | DIT 3.2 | DIT 6.4 | DT 3.2 | Total T | DI |
|---|---|---|---|---|---|---|
| | (N=97) | (N=97) | (N=98) | (N=98) | (N=390) | (N=95) |
| Patients with AEs n (%) | 91 (94) | 91 (94) | 88 (90) | 91 (93) | 361 (93) | 88 (93) |
| Not related | 41 (42) | 37 (38) | 49 (50) | 65 (66) | 192 (49) | 41 (43) |
| Related | 50 (52) | 54 (56) | 39 (40) | 26 (26) | 169 (43) | 47 (49) |
| Patients with *SAEs n (%) | 71 (73) | 67 (69) | 75 (76) | 74 (75) | 287 (74) | 72 (76) |
| Not related | 65 (67) | 61 (63) | 69 (70) | 64 (65) | 259 (66) | 59 (62) |
| Related | 6 (6) | 6 (6) | 6 (6) | 10 (10) | 28 (7) | 13 (14) |

| | | | | | | |
|---|---|---|---|---|---|---|
| *Serious Adverse Events include disease progression • Thymosin alpha 1 was well tolerated at all doses and regimens • Both arms with Thymosin 3.2 mg reached the response rate required to reject the null hypothesis | | | | | | |

### Tumor Response Data

When measured for overall tumor response, including complete response (CR) and partial response (PR), all patients in the treatment arms containing Thymosin alpha 1 showed a greater overall tumor response than those in the control arm. Patients treated with the 3.2 mg dose of Thymosin alpha 1 in combination with DTIC without interferon alpha showed an overall tumor response of 12.1%, compared to 4.1% for patients in the control group treated with DTIC and interferon alpha. While the trial was not powered to demonstrate statistical significance, the results of both arms treated with 3.2 mg of Thymosin alpha 1 were statistically significant.

**Table 12**

| Treatment Arm | | N= | Complete Response (CR) | Partial Response (PR) | Overall Response Rate (CR + PR) |
|---|---|---|---|---|---|
| DTIC + Interferon alpha (control) | | 97 | 0 | 4 | 4 (4.1%) |
| Thymosin alpha 1 (3.2 mg) + DTIC | | 99 | 2 | 10 | 12 (12.1%) |
| Thymosin alpha 1 (1.6 mg) + DTIC + Interferon alpha | | 97 | 2 | 5 | 7 (7.2%) |
| Thymosin alpha 1 (3.2 mg) + DTIC + Interferon alpha | | 97 | 3 | 7 | 10 (10.3%) |
| Thymosin alpha 1 (6.4 mg) + DTIC + Interferon alpha | | 98 | 2 | 4 | 6 (6.1%) |

### Survival Data

When measured for overall survival, all patients in the treatment arms containing Thymosin alpha 1 reached a longer median survival than those in the control arm. Patients treated with the 3.2 mg dose of Thymosin alpha 1 in combination with DTIC without interferon alpha reached a median survival of 9.3 months, compared to 6.6 months for patients in the control group treated with DTIC and interferon alpha. Progression free survival was 1.87 months for the group of patients treated with the 3.2 mg dose of Thymosin alpha 1 in combination with DTIC without interferon alpha, compared to 1.81 months for the control group treated with DTIC and interferon alpha. *Intent to treat analysis (includes all patients enrolled in the trial):*

**Table 13**

| Treatment Arm | N= | Median Survival | Median Progression Free Survival | Median Follow-up Period |
|---|---|---|---|---|
| DTIC + Interferon alpha (control) | 97 | 6.6 months | 1.81 months | 31.9 months |
| All Thymosin alpha 1 Arms | 391 | 9.4 months | 1.84 months | 26.8 months |
| Thymosin alpha 1 (3.2 mg) + DTIC | 99 | 9.3 months | 1.87 months | 32.1 months |
| Thymosin alpha 1 (1.6 mg) + DTIC + Interferon alpha | 97 | 9.3 months | 1.84 months | 28.5 months |
| Thymosin alpha 1 (3.2 mg) + DTIC + Interferon alpha | 97 | 8.5 months | 1.84 months | 28.5 months |
| Thymosin alpha 1 (6.4 mg) + DTIC + Interferon alpha | 98 | 10.2 months | 1.84 months | 17.7 months |

In a subset analysis excluding patients with elevated levels of the enzyme lactate dehydrogenase (LDH), a factor associated with poor prognosis, the group of patients with normal LDH levels treated with the 3.2 mg dose of Thymosin alpha 1 in combination with DTIC without interferon reached a median survival of 14.4 months, compared to 10.8 months for the control group treated with DTIC and interferon alpha. Progression free survival was 3.65 months for the group of normal LDH patients treated with the 3.2 mg dose of Thymosin alpha 1 in combination with DTIC without interferon alpha, compared to 2.17 months for the control group treated with DTIC and interferon alpha.

### Patients with normal lactate dehydrogenase (LDH):

**Table 14**

| Treatment Arm | N= | Median Survival | Median Progression Free Survival |
|---|---|---|---|
| DTIC + Interferon alpha (control) | 63 | 10.8 months | 2.17 months |
| All Thymosin alpha 1 Arms | 246 | 12.9 months | 3.45 months |
| Thymosin alpha 1 (3.2 mg) + DTIC. | 62 | 14.4 months | 3.65 months |
| Thymosin alpha 1 (1.6 mg) + DTIC + Interferon alpha | 62 | 12.9 months | 3.33 months |
| Thymosin alpha 1 (3.2 mg) + DTIC + Interferon alpha | 58 | 12.6 months | 3.38 months |
| Thymosin alpha 1 (6.4 mg) + DTIC + Interferon alpha | 64 | 12.8 months | 3.38 months |

Suppression of the growth of immune-sensitive tumors such as melanoma have been shown to be dependent on a strong immune response, including a large number of activated effectors such as tumor-infiltrating lymphocyte cells (TILs) and specific anti-melanoma cytotoxic T lymphocytes (CTL). It is also important to increase the presentation of cancer-specific antigens to the immune system through sustained expression of these molecules along with MHC Class I, as cancers avoid the immune system by decreases in this presentation.

Thymosin alpha 1's beneficial role in treatment of melanoma may derive from its demonstrated activation of these various arms of the immune system, including increases in TlLs, CTLs, and expression of MHC Class I and tumor-specific antigens. Thymosin alpha 1's multiple activities arise through activation of Toll-like receptor 9 and signaling through increases in the nuclear factor NfKB through Myd88 and IKKb. Evaluation of Thymosin alpha 1's utility in melanoma animal models has confirmed effective anti-tumor activity.

Example 2

Thymosin alpha 1 was able to up-regulate melanoma-specific antigens melan-A and MART-1 on the cell surface of mouse B-16 melanoma cells. The results are shown in Table 15.

**Table 15**

| Melan-A/MART-1 and Gp100 expression in B-16 mouse melanoma cells by confocal microscopy after 24h treatment with Thymosin α¹ | | | |
|---|---|---|---|
| Melanoma antigen | Control | Thymosin α1 | Thymosin α1 |
| | | 10µg/ml | 50µg/ml |
| Melan-A/MART-1 | + | + | ++ ↑ |
| Gp100 | + | + | ± ↓ |

## Claims

1. Use of an alpha thymosin peptide in a melanoma-treating effective amount in manufacture of a medicament for use in a treatment regimen for treating melanoma or a metastasis thereof in a human melanoma patient who does not have a substantially elevated LDH blood level.

2. The use of claim 1 wherein the medicament is for use in a treatment regimen which substantially excludes administration to the patient of interferon α (IFN alpha), interferon β (IFN beta), Interleukin 2 (IL-2), or a combination thereof.

3. The use of claim 1 wherein said medicament is for use in a treatment regimen which substantially excludes any immune-stimulating cytokine to said patient during said treatment regimen in an amount significant for treatment of melanoma or a metastasis thereof.

4. The use of claim 1 wherein said LDH blood level is below 475 IU/L.

5. The use of claim 4 wherein said LDH blood level is between 100 - 335 IU/L.

6. The use of claim 1 for the manufacture of a pharmaceutical combination including said alpha thymosin peptide, said combination further comprising an anti-neoplastic agent other than said alpha thymosin peptide for use during a course of the treatment regimen, which alpha thymosin peptide and other anti-neoplastic agent may be administered separately or together.

7. The use of claim 6 wherein said anti-neoplastic agent is an alkylating anti-neoplastic agent.

8. The use of claim 7 wherein said anti-neoplastic agent is dacarbazine (DTIC).

9. The use of claim 1 wherein said medicament is for use in a treatment regimen which comprises a plurality of days, said alpha thymosin peptide comprises thymosin alpha 1 (TA1), and said TA1 is for use in administration to said patient during at least a portion of said treatment regimen at a dosage within a range of 0.5 - 10 mg/day.

10. The use of claim 9 wherein said dosage is within a range of 1.5-7 mg/day.

11. The use of claim 9 wherein said dosage is within a range of 3-7 mg/day.

12. The use of claim 9 wherein said dosage is 3.2 mg/day.

13. The use of claim 9 wherein said dosage is 6.4 mg/day.

14. The use of claim 10 wherein said alpha thymosin peptide is TA1 and said medicament is for use in a treatment regimen which comprises administration of TA1 daily for a period of about 1-10 days, followed by about 1-5 days of non-administration of said TA1.

15. The use of claim 14 wherein said TA1 is for use in administration daily for about 3-5 days, followed by about 2-4 days of non-administration of said TA1.

16. The use of claim 14 wherein said TA1 is for use in administration daily for about 4 days, followed by about 3 days non-administration of said TA1.

17. The use of claim 8 wherein said dacarbazine is for administration to said patient about 7 days prior to administration of said TA1 to said patient.

18. The use of claim 17 wherein said dacarbazine is at a dosage of 700-1300 mg/m².

19. The use of claim 17 wherein said dacarbazine is at a dosage of 1000 mg/m².

20. The use of claim 9 for use in a treatment regimen which comprises administration of dacarbazine (DTIC) on a first day of treatment, followed by 6 consecutive days of non-administration of any anti-neoplastic agent to said patient, followed by 4 consecutive days of administration of TA1 to said patient, followed by 3 consecutive days of non-administration of the DTIC and TA1 to said patient, followed by 4 consecutive days of administration to said patient of TA1, and which treatment regimen further comprises repeating said treatment regimen a plurality of times with said patient, with each subsequent treatment regimen being commenced within 7-35 days from an end of a prior treatment regimen.

21. The use of claim 20 wherein each said subsequent treatment regimen is commenced within 21-28 days of the end of a prior treatment regimen.

22. The use of claim 1 wherein the alpha thymosin peptide enhances melanoma-specific surface antigens on melanoma cells.

23. The use of claim 22 wherein said antigens are melan-A, MART-1 or a combination thereof.

24. The use of claim 14 wherein the dosage rage of said TA1 is within a range of 3-7 mg/day.

25. The use of claim 20 wherein the dosage rage of said TA1 is within a range of 3-7 mg/day.
